# EUROPEAN PATENT APPLICATION

(11) **EP 3 932 916 A1**
(43) Date of publication of application: **05.01.2022**
(21) Application number: 20762529.4
(22) Date of filing: 28.02.2020
(51) Int. Cl.: C07D 407/04, C07F 7/10, G01N 21/64, A61K 49/00, C12Q 1/34

(54) **FLUORESCENT PROBE FOR DETECTING CANCER**

(30) Priority: 28.02.2019 JP 2019036670
(71) Applicant: The University of Tokyo, Bunkyo-ku, Tokyo 113-8654 (JP)
(72) Inventor: URANO Yasuteru, Tokyo 113-8654 (JP); KAMIYA Mako, Tokyo 113-8654 (JP); FUJITA Kyohei, Tokyo 113-8654 (JP)
(74) Representative: Gille Hrabal Partnerschaftsgesellschaft mbB Patentanwälte
(86) International application number: PCT/JP2020/008432
(87) International publication number: WO 2020/175688

(57) **Abstract**

To provide a fluorescent probe capable of detecting breast cancer, lung cancer, and squamous cell carcinoma.

Provided is a fluorescent probe for detecting breast cancer and a benign tumor of a mammary gland, the fluorescent probe comprising a compound represented by the following General Formula (I) or a salt thereof.

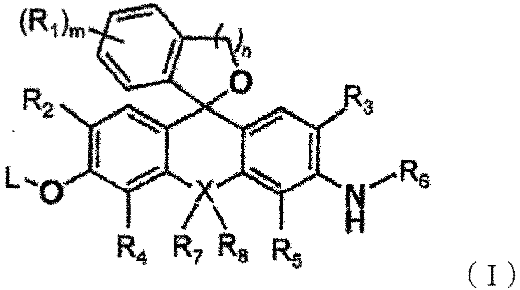

## Description

### Technical Field

The present invention relates to a fluorescent probe capable of specifically detecting cancer. More specifically, the present invention relates to a fluorescent probe capable of detecting malignant and benign tumors of the mammary gland, lung adenocarcinoma, or lung squamous cell carcinoma.

### Background Art

A method for evaluating an enzymatic activity in a malignant tissue can provide information about effective biomarkers for guiding the detection of cancer. Fluorescence-induced detection of cancer is one of the most promising approaches to improve the efficiency of a partial resection surgery. Our research group has so far developed an activated aminopeptidase-reactive fluorescent probe (Non Patent Literature 1) and successfully detected breast cancer and esophageal cancer in humans within a few minutes by locally spraying a probe solution (Non Patent Literatures 2 and 3).

However, since there is no clear difference in the aminopeptidase activity between cancer and normal tissues, there are many malignant tumors that have not yet been detected with high sensitivity and specificity by these fluorescent probes.

### Citation List

### Non Patent Literature

Non Patent Literature 1: Urano, Y. et al. Science Transl Med, 3, 110 (2011)
Non Patent Literature 2: Onoyama, H. et al. Sci. Rep., 6, 26399 (2016)
Non Patent Literature 3: Ueo, H. et al. Sci. Rep., 5, 12080 (2015)

### Summary of Invention

### Technical Problem

An object of the present invention is to provide a fluorescent probe capable of detecting malignant and benign tumors of the mammary gland, lung adenocarcinoma, or lung squamous cell carcinoma.

### Solution to Problem

By focusing on the glycosidase activity in cancer, preparing various fluorescent probes for detecting different glycosidase activities, and performing comprehensive screening of the glycosidase activity in human surgical specimens using the fluorescent probes, the present inventors have found that the glycosidase activity varies depending on the type of tumor, and certain fluorescent probes are effective for specific imaging of a tumor, thereby completing the present invention.

That is, the present invention provides the following.
[1] A fluorescent probe for detecting breast cancer and a benign tumor of a mammary gland, said fluorescent probe comprising a compound represented by the following General Formula (I) or a salt thereof, 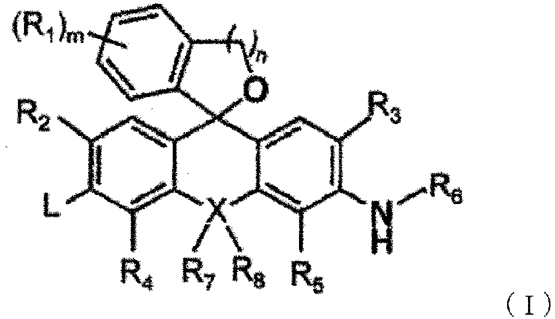 (In the formula,
   R₁, if present, represents the same or different monovalent substituents present on a benzene ring;
   R₂ and R₃ each independently represent a hydrogen atom, an alkyl group having 1 to 6 carbon atoms, or a halogen atom;
   R₄ and R₅ each independently represent a hydrogen atom, an alkyl group having 1 to 6 carbon atoms, or a halogen atom;
   R₆ represents a hydrogen atom, an alkyl group having 1 to 5 carbon atoms, or an alkyl fluoride group having 1 to 5 carbon atoms; and
   R₇ and R₈, if present, each independently represent an alkyl group having 1 to 6 carbon atoms or an aryl group,
   where R₇ and R₈ are not present in a case where X is an oxygen atom;
   X represents an oxygen atom, a silicon atom, or a carbon atom;
   n is an integer of 1 to 3; and
   L is selected from any groups of the following Formulas (1) to (6)), 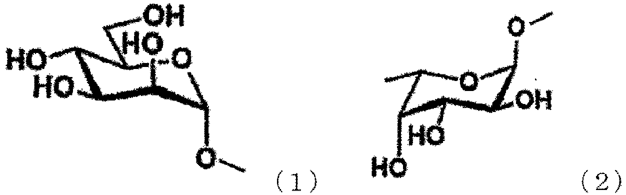 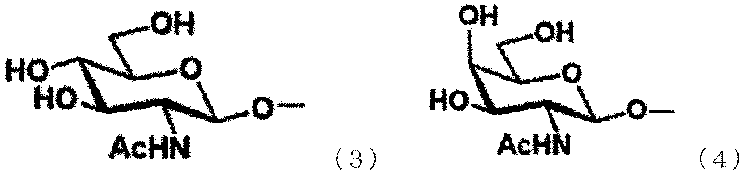 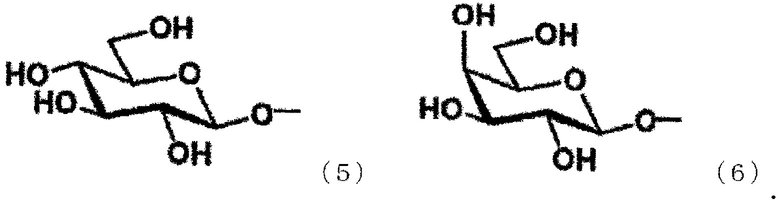
[2] The fluorescent probe according to [1], wherein X is an oxygen atom.
[3] The fluorescent probe according to [1] or [2], wherein R₆ is an alkyl fluoride group having 1 to 5 carbon atoms.
[4] A fluorescent probe for detecting breast cancer and a benign tumor of a mammary gland, said fluorescent probe comprising the fluorescent probe according to any one of [1] to [3] and a GGT-activated detection probe.
[5] A fluorescent probe for detecting lung adenocarcinoma or lung squamous cell carcinoma, said fluorescent probe comprising a compound represented by the following General Formula (I) or a salt thereof, 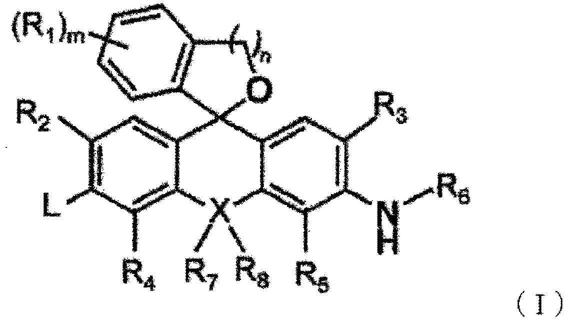 (In the formula,
   R₁, if present, represents the same or different monovalent substituents present on a benzene ring;
   R₂ and R₃ each independently represent a hydrogen atom, an alkyl group having 1 to 6 carbon atoms, or a halogen atom;
   R₄ and R₅ each independently represent a hydrogen atom, an alkyl group having 1 to 6 carbon atoms, or a halogen atom;
   R₆ represents a hydrogen atom, an alkyl group having 1 to 5 carbon atoms, or an alkyl fluoride group having 1 to 5 carbon atoms; and
   R₇ and R₈, if present, each independently represent an alkyl group having 1 to 6 carbon atoms or an aryl group,
   where R₇ and R₈ are not present in a case where X is an oxygen atom;
   X represents an oxygen atom, a silicon atom, or a carbon atom;
   n is an integer of 1 to 3; and
   L is selected from groups of the following Formula (5) or (6)), 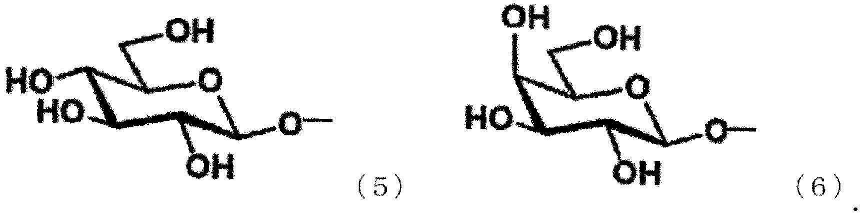
[6] The fluorescent probe according to [5], wherein X is an oxygen atom.
[7] The fluorescent probe according to [5] or [6], wherein R₆ is an alkyl fluoride group having 1 to 5 carbon atoms.
[8] A method for detecting breast cancer and a benign tumor of a mammary gland, said method comprising (a) a step of applying the fluorescent probe according to any one of [1] to [4] to a clinical breast specimen and (b) measuring a fluorescence image of the clinical breast specimen to which the fluorescent probe is applied.
[9] A method for detecting lung fiber cancer or lung squamous cell carcinoma, said method comprising (a) a step of applying the fluorescent probe according to any one of [5] to [7] to a clinical lung fiber cancer or lung squamous cell carcinoma specimen and (b) measuring a fluorescence image of the clinical lung fiber cancer or lung squamous cell carcinoma specimen to which the fluorescent probe is applied.
[10] A fluorescent probe for detecting breast cancer and a benign tumor of a mammary gland, said fluorescent probe comprising the fluorescent probe according to any one of [1] to [4] and a GGT-activated detection probe.
[11] The fluorescent probe for detecting breast cancer and a benign tumor of a mammary gland according to [10], wherein the GGT-activated detection probe is a compound represented by the following General Formula (II) or a salt thereof, 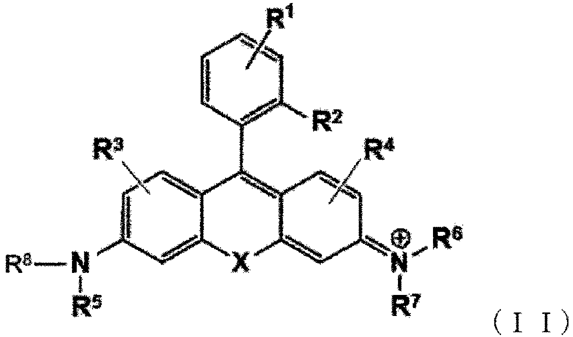 (In the formula,
   X represents Si (R^{a}) (R^{b}), Ge (R^{a}) (R^{b}), Sn(R^{a}) (R^{b}), C (R^{a}) (R^{b}), P (=O) (R^{a}), or O,
   wherein R^{a} and R^{b} each independently represent a hydrogen atom, an alkyl group, or an aryl group;
   R¹ represents a hydrogen atom or 1 to 4 same or different substituents independently selected from the group consisting of an alkyl group, a carboxyl group, an ester group, an alkoxy group, an amide group, and an azide group, which may each be substituted;
   R² represents a hydrogen atom, a hydroxyl group, a cyano group, or an alkyl group, an alkoxy group, an aryl, or a heteroaryl, which may each be substituted;
   R³ and R⁴ each independently represent a hydrogen atom or 1 to 3 same or different substituents independently selected from the group consisting of a hydroxyl group, a halogen atom, and an alkyl group, a sulfo group, a carboxyl group, an ester group, an amide group, and an azide group, which may each be substituted; and
   R⁵, R⁶, and R⁷ each independently represent a hydrogen atom or an alkyl group,
   wherein R⁶ or R⁷ may each join with R⁴ to form a ring structure containing a nitrogen atom to which R⁶ and R⁷ are bonded; and
   R⁸ represents an acyl residue derived from an amino acid).
[12] A fluorescent probe for detecting breast cancer and a benign tumor of a mammary gland, said fluorescent probe comprising the fluorescent probe according to any one of [1] to [4] and a compound of the following Formula (7) or a salt thereof, 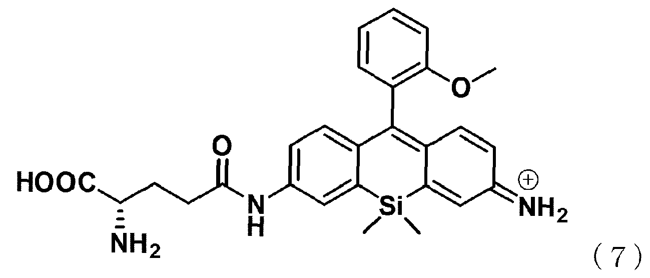
[13] A kit for detecting breast cancer and a benign tumor of a mammary gland, said kit comprising the fluorescent probe according to any one of [1] to [4].
[14] A kit for detecting breast cancer and a benign tumor of a mammary gland, said kit comprising the fluorescent probe according to any one of [1] to [4] and a GGT-activated detection probe.
[15] A kit for detecting lung fiber cancer or lung squamous cell carcinoma, said kit comprising the fluorescent probe according to any one of [5] to [7].

### Advantageous Effects of Invention

According to the present invention, it is possible to provide a fluorescent probe capable of detecting malignant and benign tumors of the mammary gland.

Furthermore, according to the present invention, it is possible to provide a fluorescent probe capable of detecting lung adenocarcinoma or lung squamous cell carcinoma.

### Brief Description of Drawings

Fig. 1 illustrates 12 types of glycosidase-reactive fluorescent probes synthesized in Examples.
Fig. 2 shows the results of a screening test of the glycosidase-reactive fluorescent probes for detecting breast cancer and benignancy.
Fig. 3 shows the results of evaluating fluorescent probes in breast cancer specimens using the glycosidase-reactive fluorescent probes (1, 2, 5, 7, 11, and 12).
Fig. 4 shows the results of comparing fluorescence increase in breast cancer and mammary fibroadenoma (benign tumor) in cases of using the glycosidase-reactive fluorescent probes 5 and 11.
Fig. 5 shows the experimental results of fluorescence imaging of breast cancer (IDC) using the 12 types of glycosidase-reactive fluorescent probes.
Fig. 6 shows the experimental results of fluorescence imaging of a benign tumor (FA) using the 12 types of glycosidase-reactive fluorescent probes.
Fig. 7 shows the experimental results of imaging mammary gland tumors using the glycosidase-reactive fluorescent probe 5 (HMRef-a-D-Man).
Fig. 8 shows the experimental results of imaging a mammary gland tumor using the glycosidase-reactive fluorescent probe 5 (HMRef-a-D-Man).
Fig. 9 shows the results of performing two-color imaging using the glycosidase-reactive fluorescent probe 5 and gGlu-2-OMeSiR600 in combination.

### Description of Embodiments

In the present specification, an "alkyl group" or an alkyl moiety of a substituent containing an alkyl moiety (for example, an alkoxy group) means, unless otherwise specified, an alkyl group having a straight chain structure, a branched chain structure, or a cyclic structure or a combination thereof having, for example, 1 to 6 carbon atoms, preferably 1 to 4 carbon atoms, and more preferably about 1 to 3 carbon atoms. More specific examples of the alkyl group can include a methyl group, an ethyl group, an n-propyl group, an isopropyl group, a cyclopropyl group, an n-butyl group, a sec-butyl group, an isobutyl group, a tert-butyl group, a cyclopropylmethyl group, an n-pentyl group, and an n-hexyl group.

In the present specification, a "halogen atom" may be any of a fluorine atom, a chlorine atom, a bromine atom, and an iodine atom, and is preferably a fluorine atom, a chlorine atom, or a bromine atom.

The present inventors focused on the glycosidase activity in cancer and prepared various fluorescent probes for detecting different glycosidase activities. Then, by utilizing the prepared fluorescent probes, the present inventors succeeded in visualizing and evaluating the intact glycosidase activity in a surgically resected cancer tissue without performing homogenization.

Specifically, the present inventors have conducted comprehensive screening of glycosidase activities in human surgical specimens, revealing that the glycosidase activity varies depending on the type of tumor and that certain fluorescent probes are effective for specific imaging of cancer. Details thereof will be described below.

### 1. Fluorescent probe for detecting breast cancer

One embodiment of the present invention is a fluorescent probe for detecting breast cancer and a benign tumor of a mammary gland, the fluorescent probe comprising a compound represented by the following General Formula (I) or a salt thereof (hereinafter, also referred to as "Embodiment 1").

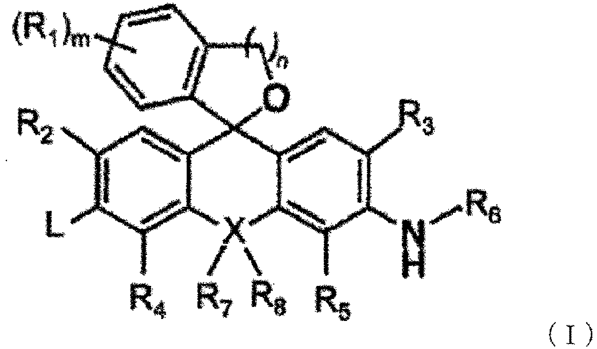

In General Formula (I), R₁, if present, represents the same or different monovalent substituents present on a benzene ring. Examples of the monovalent substituent can include a halogen and an alkyl group which may be substituted.

m is an integer of 0 to 4.

In one preferred aspect of the present invention, m is 0, and R₁ is not present. That is, the benzene ring bonded to the xanthene skeleton is an unsubstituted benzene ring.

In General Formula (I), R₂ and R₃ each independently represent a hydrogen atom, an alkyl group having 1 to 6 carbon atoms, or a halogen atom.

In a case where R₂ and R₃ represent alkyl groups, one or two or more halogen atoms, carboxy groups, sulfonyl groups, hydroxyl groups, amino groups, alkoxy groups, or the like may be present in the alkyl group, and for example, the alkyl group represented by R₂ or R₃ may be an alkyl halide group, a hydroxyalkyl group, a carboxyalkyl group, or the like. It is preferable that R₂ and R₃ are each independently a hydrogen atom or a halogen atom. In a case where R₂ and R₃ are halogen atoms, it is preferable that R₂ and R₃ are either fluorine atoms or chlorine atoms.

In one preferred aspect of the present invention, R₂ and R₃ are both hydrogen atoms.

R₄ and R₅ each independently represent a hydrogen atom, an alkyl group having 1 to 6 carbon atoms, or a halogen atom, and descriptions thereof are the same as those of R₂ and R₃. It is preferable that R₄ and R₅ are both hydrogen atoms.

R₆ represents a hydrogen atom, an alkyl group having 1 to 5 carbon atoms, or an alkyl fluoride group having 1 to 5 carbon atoms. The alkyl group represented by R₆ is preferably a methyl group or an ethyl group. The alkyl fluoride group represented by R₆ is preferably -CH₂-CF₃ or - CH₂-CH₂-CF₃.

In one preferred aspect of the present invention, R₆ is -CH₂-CF₃.

In General Formula (I), R₇ and R₈, if present, each independently represent an alkyl group having 1 to 6 carbon atoms or an aryl group, and it is preferable that R₇ and R₈ are each independently an alkyl group having 1 to 3 carbon atoms and more preferable that R₇ and R₈ are both methyl groups. One or two or more halogen atoms, carboxy groups, sulfonyl groups, hydroxyl groups, amino groups, alkoxy groups, or the like may be present in the alkyl group represented by R₇ and R₈, and for example, the alkyl group represented by R₇ or R₈ may be an alkyl halide group, a hydroxyalkyl group, a carboxyalkyl group, or the like.

In a case where R₇ or R₈ represents an aryl group, the aryl group may be any of a monocyclic aromatic group or a fused aromatic group, and the aryl ring may contain one or two or more ring-constituting heteroatoms (for example, a nitrogen atom, an oxygen atom, a sulfur atom, or the like). The aryl group is preferably a phenyl group. One or two or more substituents may be present on the aryl ring. As the substituent, for example, one or two or more halogen atoms, carboxy groups, sulfonyl groups, hydroxyl groups, amino groups, alkoxy groups, or the like may be present.

Furthermore, in a case where X described below is an oxygen atom, R₇ and R₈ are not present.

X represents an oxygen atom, a silicon atom, or a carbon atom.

In one preferred aspect of the present invention, X is an oxygen atom.

n is an integer of 1 to 3 and is preferably 1.

In General Formula (I), L is selected from any groups of the following Formulas (1) to (6).

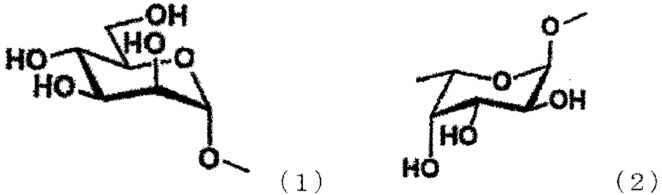

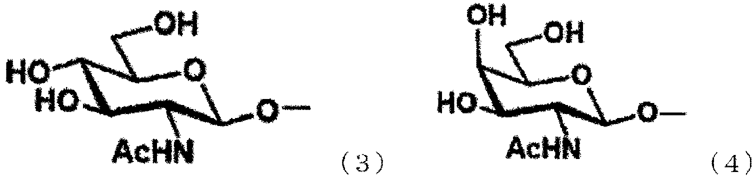

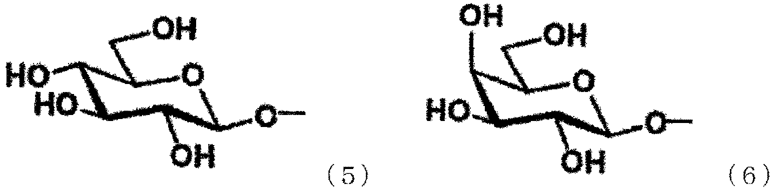

A fluorescent probe having a group of Formula (1) as L is an α-mannosidase-reactive fluorescent probe, a fluorescent probe having a group of Formula (2) as L is an α-L-fucosidase-reactive fluorescent probe, a fluorescent probe having a group of Formula (3) as L is a β-hexosaminidase-reactive fluorescent probe, a fluorescent probe having a group of Formula (4) as L is a β-N-acetylgalactosaminidase-reactive fluorescent probe, a fluorescent probe having a group of Formula (5) as L is a β-glucosidase-reactive fluorescent probe, and a fluorescent probe having a group of Formula (6) as L is a β-galactosidase-reactive fluorescent probe. According to the results of study by the present inventors, these fluorescent probes were effective for diagnostic imaging of mammary fibroadenoma, invasive ductal carcinoma, and ductal carcinoma in-situ. Therefore, the fluorescent probe of the present invention having any group of Formulas (1) to (6) as L is capable of detecting both breast cancer and a benign tumor.

One preferred aspect of Embodiment 1 of the present invention is the fluorescent probe for detecting breast cancer and a benign tumor of a mammary gland, the fluorescent probe comprising a compound represented by the following Formula (Ia) or a salt thereof.

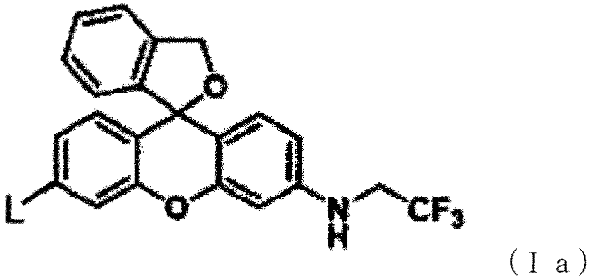

Here, L is selected from any groups of the following Formulas (1) to (6).

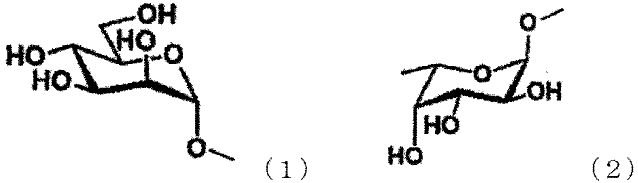

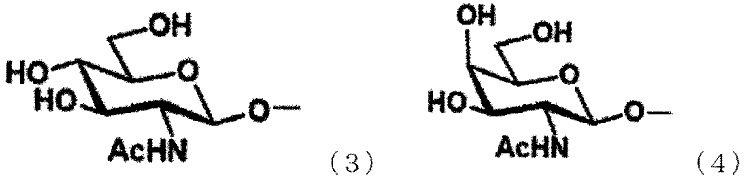

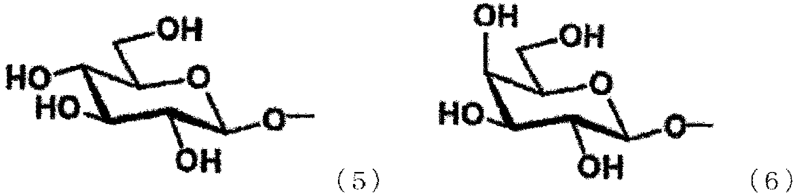

### 2. Fluorescent probe for detecting lung cancer or squamous cell carcinoma

Another embodiment of the present invention is a fluorescent probe for detecting lung fiber cancer or lung squamous cell carcinoma, the fluorescent probe comprising a compound represented by the following General Formula (I) or a salt thereof (hereinafter, also referred to as "Embodiment 2").

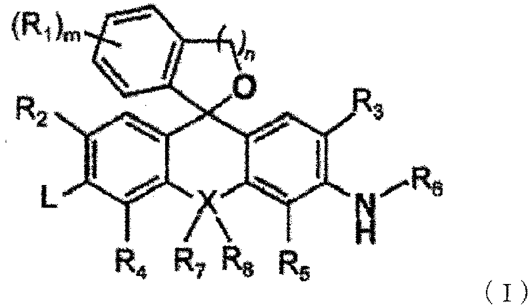

In General Formula (I), R₁ to R₈, X, and n are the same as those described in Embodiment 1.

In Embodiment 2, L is selected from groups of the following Formula (5) or (6).

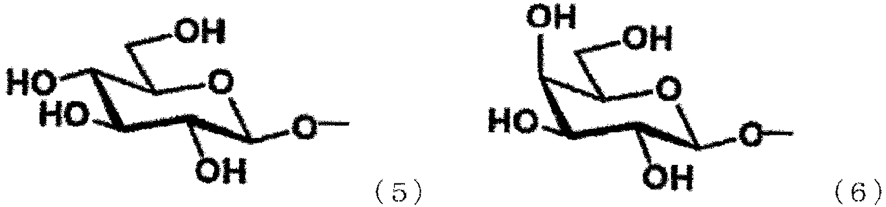

A fluorescent probe having a group of Formula (5) or (6) as L is a β-glucosidase- or β-galactosidase-reactive fluorescent probe, and according to the results of study by the present inventors, these fluorescent probes were found to be effective for specific imaging of main lung cancer including lung adenocarcinoma in addition to lung squamous cell carcinoma that could not be detected by the aminopeptidase-reactive fluorescent probe. Therefore, the fluorescent probe of the present invention having a group of Formula (5) or (6) as L is capable of specifically detecting lung adenocarcinoma or lung squamous cell carcinoma.

One preferred aspect of Embodiment 2 of the present invention is the fluorescent probe for detecting lung fiber cancer or lung squamous cell carcinoma, the fluorescent probe comprising a compound represented by the following Formula (Ia) or a salt thereof.

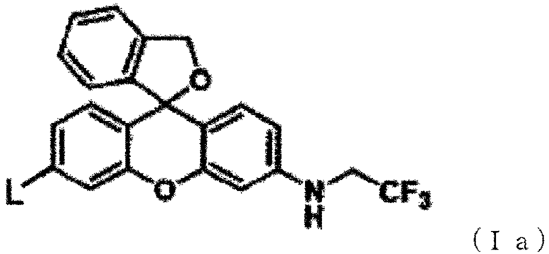

Here, L is selected from groups of the following Formula (5) or (6).

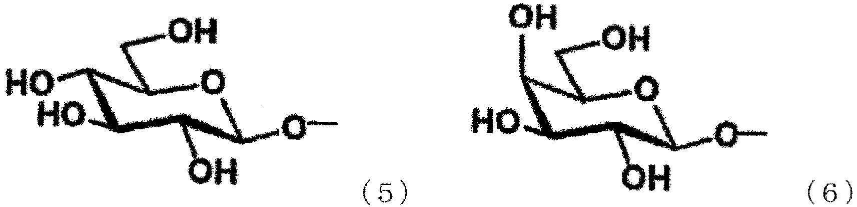

The compound represented by General Formula (I) in Embodiments 1 and 2 can exist as an acid addition salt or a base addition salt. Examples of the acid addition salt can include a mineral acid salt such as hydrochloride, sulfate, and nitrate or an organic acid salt such as methanesulfonate, p-toluenesulfonate, oxalate, citrate, and tartrate, and examples of the base addition salt can include a metal salt such as a sodium salt, a potassium salt, a calcium salt, and a magnesium salt, an ammonium salt, or an organic amine salt such as a triethylamine salt. In addition to these, a salt may be formed with an amino acid such as glycine. The compound represented by General Formula (I) or a salt thereof may also exist as a hydrate or a solvate, and these substances can also be used in the present invention.

The compound represented by General Formula (I) may have one or two or more asymmetric carbons depending on the type of the substituent, and in addition to a stereoisomer such as an optically active substance based on one or two or more asymmetric carbons and a diastereoisomer based on two or more asymmetric carbons, an arbitrary mixture of stereoisomers, a racemate, and the like can also be used in the present invention.

Methods for producing representative compounds of the compound represented by General Formula (I) were specifically presented in Examples of the present specification. Therefore, those skilled in the art can produce the compound represented by General Formula (I) by appropriately selecting reaction raw materials, reaction conditions, reaction reagents, and the like based on these descriptions and modifying or changing the methods as necessary.

### 3. Fluorescent probe used in combination with GGT-activated detection probe

The fluorescent probe of Embodiment 1 can be used in combination with a GGT-activated detection probe. For example, when a red GGT probe and the fluorescent probe of Embodiment 1 are used in combination for the detection of breast cancer, red fluorescence is similarly emitted in both breast cancer (malignant tumor) and a benign tumor, whereas the glycosidase-reactive fluorescent probe used in the present invention emits strong green fluorescence particularly in a benign tumor. When the two images were combined, it was possible to image the breast cancer tissue in red and the benign tumor tissue in yellow. Therefore, by using the glycosidase-reactive fluorescent probe and the GGT probe in combination, it becomes possible to determine that a site where only the glycosidase-reactive fluorescent probe fluoresces is a benign tumor site, and a site where both the glycosidase-reactive fluorescent probe and the GGT probe fluoresce is a breast cancer (malignant tumor) site, and it becomes possible to distinguish between the benign tumor and the malignant tumor. Thus, the efficiency of a partial resection surgery can be improved without performing resection of the benign tumor site in a surgical procedure.

That is, another embodiment of the present invention is a fluorescent probe for detecting breast cancer and a benign tumor of a mammary gland, the fluorescent probe comprising the fluorescent probe of Embodiment 1 and a GGT-activated detection probe.

Here, examples of the GGT-activated detection probe can include a compound represented by the following General Formula (II) or a salt thereof.

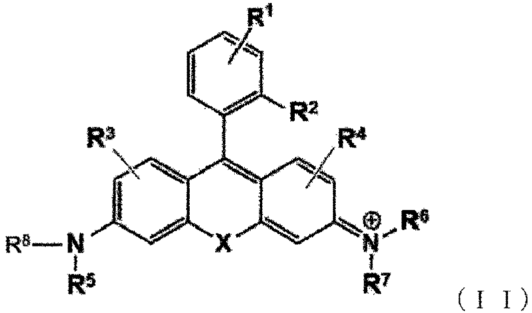

In General Formula (II), X represents Si (R^{a}) (R^{b}), Ge(R^{a})(R^{b}), Sn(R^{a})(R^{b}), C(R^{a})(R^{b}), P(=O)(R^{a}), or O. Here, R^{a} and R^{b} each independently represent a hydrogen atom, an alkyl group, or an aryl group.

R¹ represents a hydrogen atom or 1 to 4 same or different substituents independently selected from the group consisting of an alkyl group, a carboxyl group, an ester group, an alkoxy group, an amide group, and an azide group, which may each be substituted.

R² represents a hydrogen atom, a hydroxyl group, a cyano group, or an alkyl group, an alkoxy group, an aryl, or a heteroaryl, which may each be substituted.

R³ and R⁴ each independently represent a hydrogen atom or 1 to 3 same or different substituents independently selected from the group consisting of a hydroxyl group, a halogen atom, and an alkyl group, a sulfo group, a carboxyl group, an ester group, an amide group, and an azide group, which may each be substituted.

R⁵, R⁶, and R⁷ each independently represent a hydrogen atom or an alkyl group.

Here, R⁶ or R⁷ may each join with R⁴ to form a ring structure containing a nitrogen atom to which R⁶ and R⁷ are bonded.

R⁸ represents an acyl residue derived from an amino acid. Here, the acyl residue refers to a residue which is a partial structure that remains after an OH group is removed from a carboxyl group of an amino acid. That is, the carbonyl moiety of the acyl residue derived from an amino acid and NH adjacent to R⁸ of Formula (II) form an amide bond, whereby the acyl residue is linked to the rhodamine skeleton.

As the "amino acid", any compound can be used as long as it is a compound having both an amino group and a carboxyl group, including natural and non-natural compounds. The amino acid may be any of a neutral amino acid, a basic amino acid, and an acidic amino acid. In addition to an amino acid that itself functions as a transmitter such as a neurotransmitter, an amino acid that is a constituent of a physiologically active peptide (including a dipeptide, a tripeptide, a tetrapeptide, and an oligopeptide) or a polypeptide compound such as a protein can be used. For example, the amino acid may be an α amino acid, a β amino acid, a γ amino acid, or the like. As the amino acid, an optically active amino acid is preferably used. For example, as the α amino acid, either D- or L-amino acid may be used, however, it may be preferable to select an optically active amino acid that functions in a living body.

R⁸ is a site that is cleaved by a reaction with a target peptidase. The target peptidase can be γ-glutamyl transpeptidase (GGT), dipeptidyl peptidase IV (DPP-IV), or calpain. Therefore, when the target peptidase is γ-glutamyl transpeptidase, R⁸ is preferably a γ-glutamyl group. Furthermore, when the target peptidase is dipeptidyl peptidase IV, R⁸ is preferably an acyl group containing a proline residue. When the target peptidase is calpain, R⁸ can be, for example, an acyl group containing a cysteine residue, or Suc-Leu-Leu-Val-Tyr (Suc-LLVY) or AcLM known in the technical field as a calpain substrate can also be used.

For the principle of the fluorescence emission by the compound represented by General Formula (II), refer to Japanese Patent Application No. 2018-210101, which is a pending application.

One preferred aspect of the present invention is the fluorescent probe for detecting breast cancer and a benign tumor of a mammary gland, the fluorescent probe comprising the fluorescent probe of Embodiment 1 and a compound of General Formula (II) in which R⁸ is a γ-glutamyl group or a salt thereof.

Among the compounds represented by General Formula (II), the following compound can be preferably used.

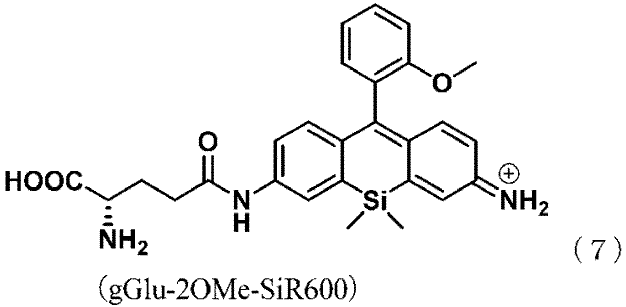

The compound represented by General Formula (II) can exist as an acid addition salt or a base addition salt. Examples of the acid addition salt can include a mineral acid salt such as hydrochloride, sulfate, and nitrate or an organic acid salt such as methanesulfonate, p-toluenesulfonate, oxalate, citrate, and tartrate, and examples of the base addition salt can include a metal salt such as a sodium salt, a potassium salt, a calcium salt, and a magnesium salt, an ammonium salt, or an organic amine salt such as a triethylamine salt. In addition to these, a salt may be formed with an amino acid such as glycine. The compound represented by General Formula (II) or a salt thereof may also exist as a hydrate or a solvate, and these substances can also be used in the present invention.

The compound represented by General Formula (II) may have one or two or more asymmetric carbons depending on the type of the substituent, and in addition to a stereoisomer such as an optically active substance based on one or two or more asymmetric carbons and a diastereoisomer based on two or more asymmetric carbons, an arbitrary mixture of stereoisomers, a racemate, and the like can also be used in the present invention.

One preferred aspect of the present invention is the fluorescent probe for detecting breast cancer and a benign tumor of a mammary gland, the fluorescent probe comprising the fluorescent probe of Embodiment 1 and a compound of Formula (7) or a salt thereof.

### 4. Kit for detecting breast cancer and benign tumor of mammary gland using fluorescent probe of present invention

Another embodiment of the present invention is a kit for detecting breast cancer and a benign tumor of a mammary gland, the kit comprising the fluorescent probe of Embodiment 1.

Another embodiment of the present invention is a kit for detecting breast cancer and a benign tumor of a mammary gland, the kit comprising the fluorescent probe of Embodiment 1 and a GGT-activated detection probe. The GGT-activated detection probe is as described above.

Another embodiment of the present invention is a kit for detecting lung fiber cancer or lung squamous cell carcinoma, the kit comprising the fluorescent probe of Embodiment 2.

In the kit, the fluorescent probe of the present invention is generally prepared as a solution, however, the fluorescent probe of the present invention can also be provided as, for example, a composition in an appropriate form such as a mixture in a powder form, a lyophilizate, a granule, a tablet, and a liquid and can be dissolved in distilled water for injection or an appropriate buffer at the time of use to be applied.

In addition, the kit may appropriately contain an additional reagent or the like as necessary. For example, an additive such as a solubilizing agent, a pH adjusting agent, a buffering agent, and an isotonizing agent can be used as the additive, and the blending amount thereof can be appropriately selected by those skilled in the art.

Another embodiment of the present invention is a method for detecting breast cancer and a benign tumor of a mammary gland, the method comprising (a) a step of applying the fluorescent probe of Embodiment 1 to a clinical breast specimen and (b) measuring a fluorescence image of the clinical breast specimen to which the fluorescent probe is applied.

The application of the fluorescent probe to the clinical breast specimen in the step of (a) can be performed, for example, by locally spraying a solution of the fluorescent probe to the clinical breast specimen.

Another embodiment of the present invention is a method for detecting lung fiber cancer or lung squamous cell carcinoma, the method comprising (a) a step of applying the fluorescent probe of Embodiment 2 to a clinical lung fiber cancer or lung squamous cell carcinoma specimen and (b) measuring a fluorescence image of the clinical lung fiber cancer or lung squamous cell carcinoma specimen to which the fluorescent probe is applied.

The application of the fluorescent probe to the clinical lung fiber cancer or lung squamous cell carcinoma specimen in the step of (a) can be performed, for example, by locally spraying a solution of the fluorescent probe to the clinical lung fiber cancer or lung squamous cell carcinoma specimen.

### [Examples]

Hereinafter, the present invention will be described using Examples, but the present invention is not limited thereto.

### [Synthesis Example 1]

### Synthesis of probe 1 (β-glucosidase-reactive probe)

HMRef (0.426 g, 1.07 mmol), 2,3,4,6-tetra-O-acetyl α-D-glucopyranosyl bromide (8.26 g, 20.1 mmol), Ag₂O (4.71 g, 20.3 mmol), and Na₂SO₄ (720 mg, 2.00 mmol) were dissolved in 30 mL of acetonitrile (super dehydrated), and the mixture was stirred at room temperature for 24 hours. The reaction solution was filtered through Celite, the filtrate was collected, and acetonitrile was removed under reduced pressure. The residue was purified by silica gel column chromatography (CH₂Cl₂:MeOH = 95:5) to obtain an acetylated sugar adduct. The acetylated sugar adduct was dissolved in methanol, a solution obtained by dissolving NaOMe (520 mg, 9.63 mmol) in 5 mL of methanol was added thereto, and the mixture was stirred at room temperature for 5 hours. The reaction solution was neutralized with Amberlite IR 120, and methanol was removed under reduced pressure. The residue was purified by silica gel column chromatography (CH₂Cl₂:MeOH = 90:10) to obtain a target product (301 mg, 0.535 mmol) in 50.2% yield.

### [Synthesis Example 2]

### Synthesis of probe 5 (a-mannosidase-reactive probe)

HMRef (96.0 mg, 0.24 mmol), penta-O-acetyl-D-mannopyranoside (3.33 g, 8.53 mmol), boron trifluoride diethyl ether complex (8.0 mL, 63.4 mmol), and Na₂SO₄ (500 mg, 3.34 mmol) were dissolved in 15 mL of dichloromethane, and the mixture was stirred for 24 hours. The reaction solution was filtered through Celite, and the dichloromethane layer was extracted by liquid separation using 1 M NaOH and removed under reduced pressure. The residue was dissolved in 15 mL of methanol, a solution obtained by dissolving NaOMe (1.60 g, 29.6 mmol) in 5 mL of methanol was added thereto, and the mixture was stirred at room temperature for 5 hours. The reaction solution was neutralized with Amberlite IR 120, and methanol was removed under reduced pressure. The residue was purified by silica gel column chromatography (CH₂Cl₂:MeOH = 90:10) to obtain a crude product. The crude product was further purified by HPLC (5 to 80% MeCN in water (0.1% TFA) over 90 min) to obtain a target product (61.6 mg, 0.200 mmol) in 45.6% yield.

### [Synthesis Example 3]

### Synthesis of probe 7 (a-L-fucosidase-reactive probe)

HMRef (90.0 mg, 0.226 mmol), 2,3,4-tri-O-acetyl-L-fucopyranosyl trichloroacetimidate (862 mg, 1.99 mmol), and TMSOTf (442 mg, 1.99 mmol) were dissolved in 10 mL of dichloromethane, and the mixture was stirred at -41°C for 12 hours. The reaction solution was diluted with dichloromethane and neutralized with a saturated aqueous sodium bicarbonate solution. The organic layer was extracted by liquid separation and removed under reduced pressure. The residue was purified by silica gel column chromatography (CH₂Cl₂:MeOH = 95:5) to obtain an intermediate. The intermediate was dissolved in 5 mL of methanol, a solution obtained by dissolving NaOMe (1.33 g, 24.6 mmol) in 5 mL of methanol was added thereto, and the mixture was stirred at room temperature for 5 hours. The reaction solution was neutralized with Amberlite IR 120, and methanol was removed under reduced pressure. The residue was purified by silica gel column chromatography (CH₂Cl₂:MeOH = 95:5) to obtain a crude product. The crude product was further purified by HPLC (5 to 80% MeCN in water (0.1% TFA) over 90 min) to obtain a target product (14.4 mg, 0.0257 mmol) in 11.7% yield.

### [Synthesis Example 4]

### Synthesis of probe 12 (β-N-acetylgalactosaminidase-reactive probe)

HMRef (46.7 mg, 0.117 mmol), 2-acetoamide-3,4,6-triO-acetyl-2-deoxy-a-D-galactopyranosyl chloride (400 mg, 1.09 mmol), Ag₂O (400 mg, 1.72 mmol), NaI (88.3 mg, 0.589 mmol), and Na₂SO₄ (500 mg, 3.34 mmol) were dissolved in 10 mL of acetonitrile (super dehydrated), and the mixture was stirred at room temperature for 24 hours. The reaction solution was filtered through Celite, the filtrate was collected, and acetonitrile was removed under reduced pressure. The residue was purified by silica gel column chromatography (CH₂Cl₂:MeOH = 95:5) to obtain an acetylated sugar adduct. The acetylated sugar adduct was dissolved in methanol, a solution obtained by dissolving NaOMe (0.50 g, 9.25 mmol) in 5 mL of methanol was added thereto, and the mixture was stirred at room temperature for 5 hours. The reaction solution was neutralized with Amberlite IR 120, and methanol was removed under reduced pressure. The residue was purified by silica gel column chromatography (CH₂Cl₂:MeOH = 90:10) to obtain a crude product. The crude product was further purified by HPLC (5 to 80% MeCN in water (0.1% TFA) over 90 min) to obtain a target product (11.3 mg, 0.0216 mmol) in 28.2% yield.

A probe 2 was synthesized according to the description of Matsuzaki, H et al, Bioconju. Chem., 27(4), 973-981 (2016), and a probe 11 was synthesized according to the description of Asanuma, D et al, Nat. Commun., 6, 6463 (2015).

Furthermore, probes 3, 4, 6, 8, 9, and 10 were synthesized in the same manner as the above probe.

### [Example 1]

### Screening of glycosidase-reactive fluorescent probes for detecting breast cancer and benignancy

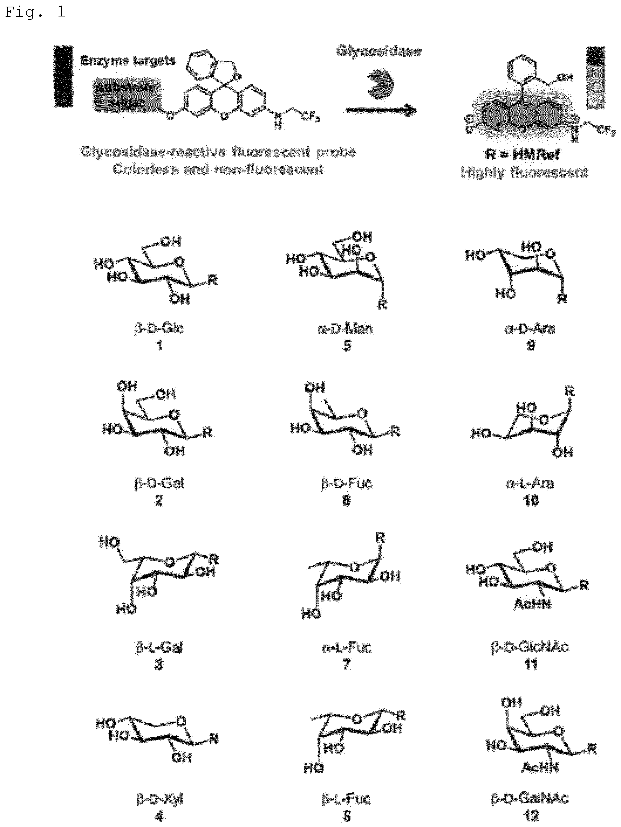

Using the 12 types of glycosidase-reactive fluorescent probes synthesized above (refer to Fig. 1), screening for the detection of breast cancer and benignancy was performed in the following procedure.

200 µL of a PBS solution of each fluorescent probe at a concentration of 50 µM was added to each well in which a clinical mammary gland specimen was placed, and fluorescence images at 1, 3, 5, 10, 20, and 30 minutes were acquired at 540 nm using a Maestro in vivo imaging system (PerkinElmer Inc.). Each fluorescence intensity value was quantified and compared by taking Regions Of Interest (ROIs) on Maestro software. Ex/Em = 465 - 30 nm/515 nm long-pass was used as the filter setting.

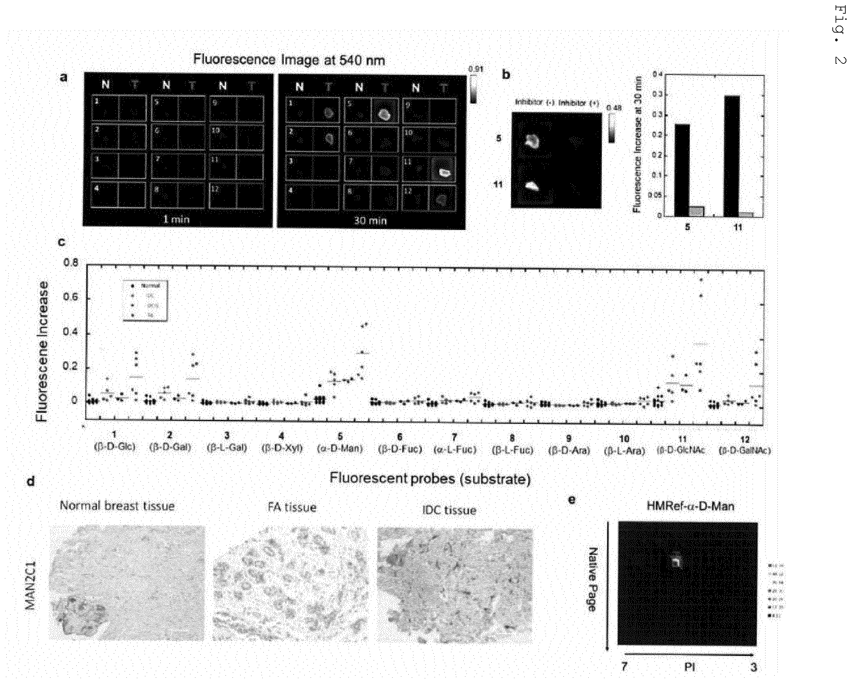

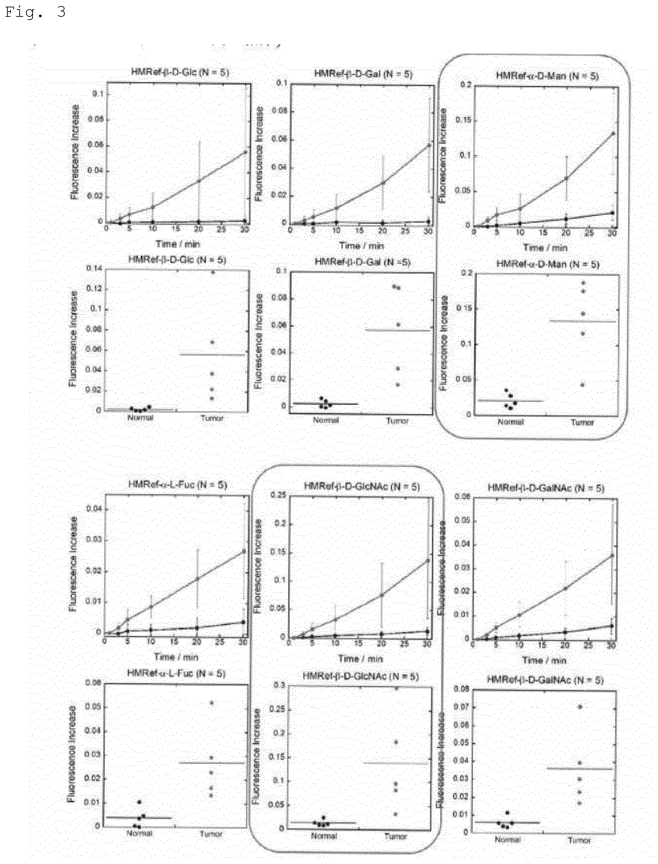

The results are shown in Fig. 2.
a of Fig. 2 shows the results of the screening of the fluorescent probes using a surgically resected human breast specimen.
b of Fig. 2 shows fluorescence increase at 30 minutes in a breast FA tissue in the presence and absence of each inhibitor. The black bar graph represents the increase in fluorescence in the absence of the inhibitor and the gray bar graph represents the increase in fluorescence in the presence of the inhibitor. The fluorescent probe concentration was 50 µM, and the inhibitor concentration was 500 µM.
c of Fig. 2 shows the results of comprehensive analysis of intact glycosidase activities in normal breast, IDC (breast cancer), and FA (benign tumor) tissues using the 12 types of fluorescent probes. The increase in fluorescence indicates increase after the lapse of 1 minute to 30 minutes since the addition of the fluorescent probe. Each dot represents, from left to right, increase in fluorescence in normal breast, IDC, DCIS, and FA tissues. In c of Fig. 3, the glycosidase-reactive fluorescent probes 1, 2, 5, 7, 11, and 12 showed increase in fluorescence in both the breast cancer and FA (benign tumor) tissues.
d of Fig. 2 shows the results of immunohistochemical analysis of normal mammary gland, FA, or IDC tissue for MAN2C1. If no stained cells were found, the tissue was evaluated as negative, otherwise the tissue was evaluated as positive. As a result, stronger staining was confirmed in the FA and IDC tissues than in the normal tissue, and overexpression of MAN2C1 was confirmed in these tissues.
e of Fig. 2 shows the results of a DEG assay. The DEG assay was performed according to the procedure described in J. Am. Chem. Soc., 135, 6002-6005 (2013).

As a result of performing the DEG assay on the FA tissue using the α-mannosidase-reactive probe 5, only one fluorescence spot was observed by two-dimensional electrophoresis, and MAN2C1 was identified from the spot by peptide mass fingerprinting. Also in the IDC tissue, only one similar fluorescence spot was observed by two-dimensional electrophoresis. From these results, it was found that the responsible enzyme involved in the increase in fluorescence of the α-mannosidase-reactive probe 5 was MAN2C1.

Evaluation of a fluorescent probe in a breast cancer specimen was performed on the six glycosidase-reactive fluorescent probes (1, 2, 5, 7, 11, and 12) that showed increased fluorescence in c of Fig. 2. As the specimen, 4 cases of breast cancer IDC and 1 case of DCIS were used, and the fluorescent probe concentration was 50 µM.

The results are shown in Fig. 3. From Fig. 3, it was confirmed that the increase in fluorescence of the glycosidase-reactive fluorescent probes 5 and 11 was large, and fluorescence intensity ratios between the tumor and the normal tissues were also large for these probes.

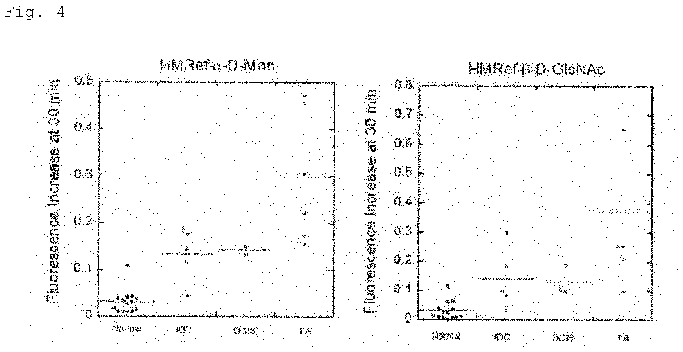

The results of comparing the increase in fluorescence of the glycosidase-reactive fluorescent probes 5 (HMRef-α-D-Man) and 11 (HMRef-β-D-GlcNAc) in breast cancer and the benign tumor are shown in Fig. 4 (fluorescent probe concentration = 50 µM).

200 µL of a PBS solution of each fluorescent probe at the above concentration was added to each well in which a clinical mammary gland specimen was placed, and fluorescence images at 1, 3, 5, 10, 20, and 30 minutes were acquired at 540 nm using the Maestro in vivo imaging system (PerkinElmer Inc.). Each fluorescence intensity value was quantified and compared by taking Regions Of Interest (ROIs) on Maestro software. Ex/Em = 465 - 30 nm/515 nm long-pass was used as the filter setting.

As a result, the glycosidase-reactive fluorescent probes 5 (HMRef-α-D-Man) and 11 (HMRef-β-D-GlcNAc) showed higher fluorescence increase in the tumor tissue as compared with the normal mammary gland (Normal), and showed higher fluorescence in the benign tumor tissue (FA) than in the cancer tissues (IDC and DCIS). These results suggest that both probes may be effective for breast cancer fluorescence imaging or specific detection of benign tumors.

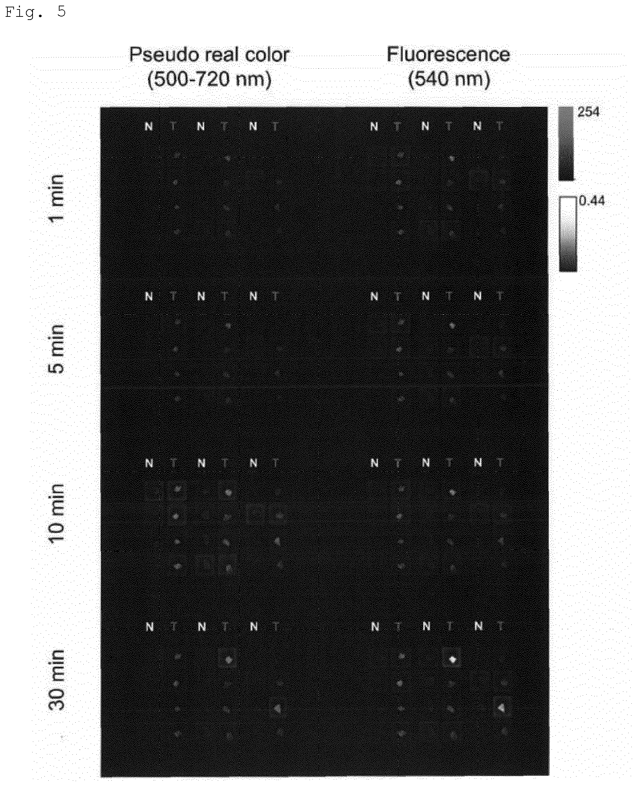

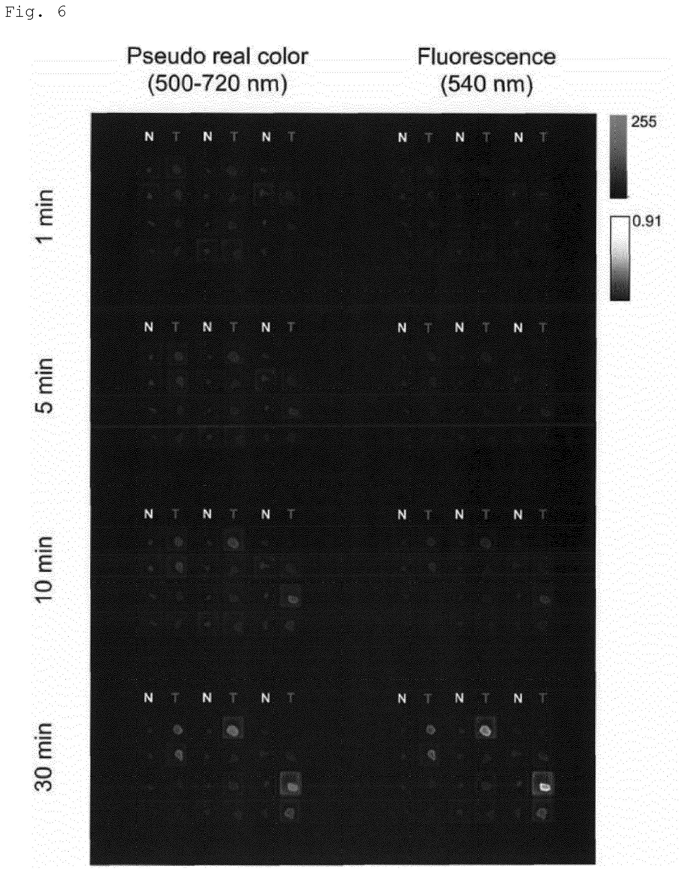

Figs. 5 and 6 show images acquired by imaging temporal changes in fluorescence increase in breast cancer (IDC) and the benign tumor (FA) using the 12 types of glycosidase-reactive fluorescent probes. The experiment was performed under the following conditions.

200 mL of a PBS solution of each fluorescent probe at the above concentration was added to each well in which a clinical mammary gland specimen was placed, and fluorescence images at 1, 3, 5, 10, 20, and 30 minutes were acquired at 540 nm using the Maestro in vivo imaging system (PerkinElmer Inc.). Each fluorescence intensity value was quantified and compared by taking Regions Of Interest (ROIs) on Maestro software. Ex/Em = 465 - 30 nm/515 nm long-pass was used as the filter setting.

Fig. 5 is a result of fluorescence imaging using the breast cancer tissue (right figure), and Fig. 6 is a result of fluorescence imaging using the benign tumor (FA) (right figure) .

### [Example 2]

### Mammary gland tumor imaging using HMRef-a-D-Man

Using the glycosidase-reactive fluorescent probe 5 (HMRef-α-D-Man) (concentration: 50 mM), imaging was performed under the following conditions.

3 mL of a PBS solution of the fluorescent probe at the above concentration was added to a dish on which a clinical mammary gland specimen (specimen in which a normal part and a tumor part were mixed) was placed, and a fluorescence image at each time was acquired. Hereinafter, fluorescence images of clinical DCIS and FA specimens were acquired using the Maestro in vivo imaging system (PerkinElmer Inc.). Ex/Em = 465 - 30 nm/515 nm long-pass was used as each filter setting. Fluorescence images of the clinical IDC specimen were acquired using a homme made portable imaging apparatus equivalent to the Maestro in vivo imaging system.

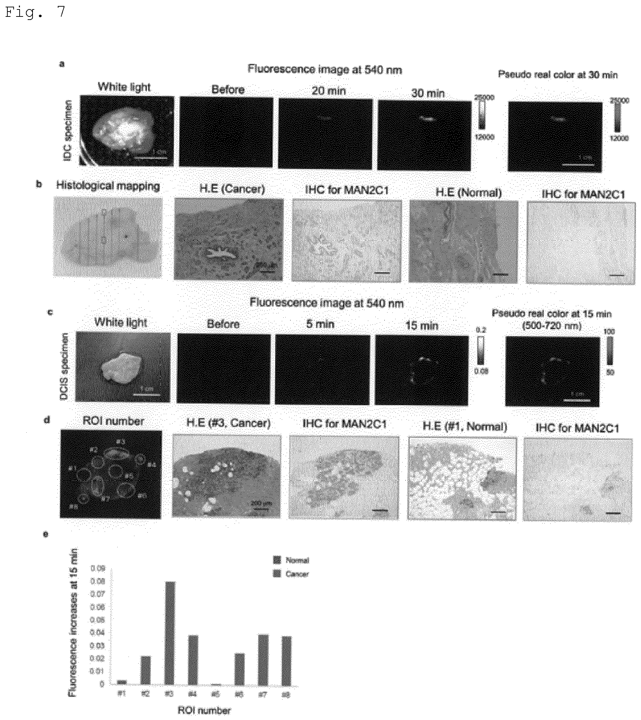

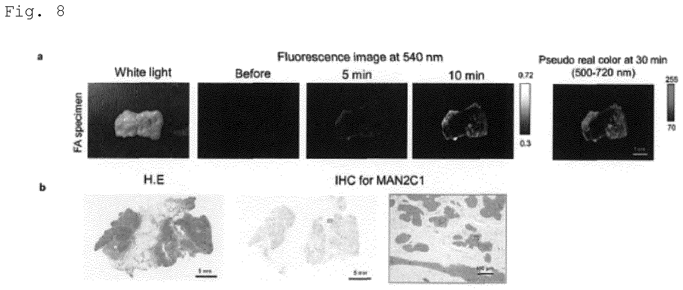

The results are shown in Figs. 7 and 8.
a of Fig. 7 shows results of imaging surgically resected IDC (breast cancer).
   After spraying the probe, only the IDC site was visualized with strong fluorescence in about 20 minutes.
b of Fig. 7 shows the results of histological analysis of the specimens and the results of immunostaining MAN2C1. It was confirmed that the site where increase in fluorescence was observed coincided with the site that was histologically IDC and the site where MAN2C1 was highly expressed.
c of Fig. 7 shows results of imaging surgically resected DCIS (breast cancer). After spraying the probe, only the DCIS site was visualized with strong fluorescence within 15 minutes.
d of Fig. 7 shows an example of Regions Of Interest (ROIs) of the specimens, the results of histological analysis of the specimens, and the results of immunostaining MAN2C1. It was confirmed that the site where increase in fluorescence was observed coincided with the site that was histologically DCIS and the site where MAN2C1 was highly expressed. In addition, detection of a very minute DCIS tissue of 1 mm or less was also confirmed.
e of Fig. 7 shows fluorescence increase values at Regions Of Interest (ROIs) of the specimens at 15 minutes. No significant increase in fluorescence was observed at the sites that were histologically normal, and significant increase in fluorescence was observed at the sites that were histologically DCIS.

a of Fig. 8 shows results of imaging the surgically resected FA (benign tumor). After spraying the probe, only the FA site was visualized with strong fluorescence within 10 minutes.
b of Fig. 8 shows the result of histological analysis of the specimen and the results of immunostaining MAN2C1. It was confirmed that the site where increase in fluorescence was observed coincided with the site that was histologically FA and the site where MAN2C1 was highly expressed.

### [Example 3]

### Distinguishing between benign tumor and breast cancer using HMRef-α-D-Man and GGT probe in combination

Two-color imaging was performed under the following conditions using the glycosidase-reactive fluorescent probe 5 (HMRef-α-D-Man) (concentration: 50 µM) and a GGT probe gGlu-2-OMeSiR600 (concentration: 50 µM) in combination.

200 µL of a PBS solution of the fluorescent probes at the above concentrations was added to each well in which a clinical mammary gland specimen was placed, and fluorescence images at 1, 3, 5, 10, 20, and 30 minutes were acquired at 540 nm and 640 nm using the Maestro in vivo imaging system (PerkinElmer Inc.). Each fluorescence intensity value was quantified and compared by taking Regions Of Interest (ROIs) on Maestro software. For the glycosidase-reactive fluorescent probe 5, Ex/Em = 465 - 30 nm/515 nm long-pass was used as the filter setting. For the GGT probe, Ex/Em = 570 - 40 nm/610 nm long-pass was used.

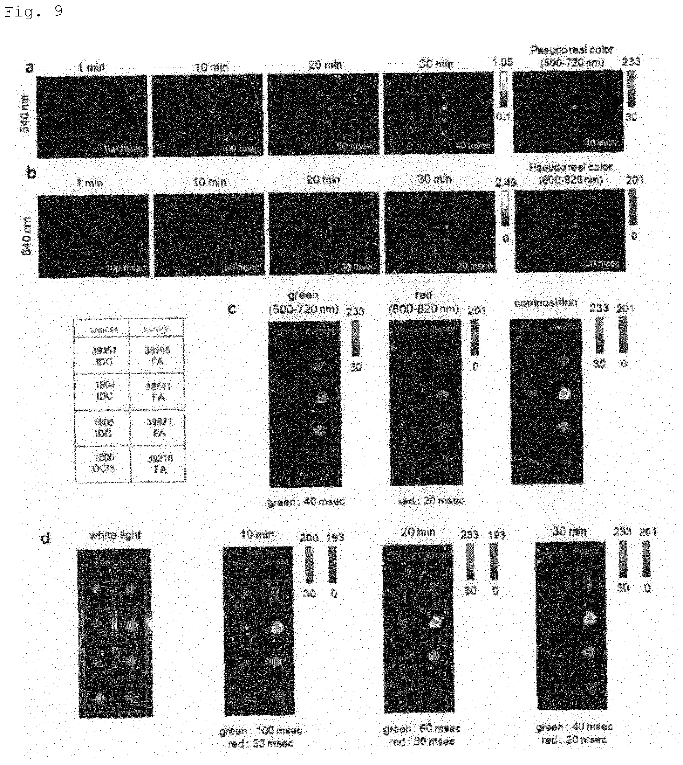

The results are shown in Fig. 9.
a of Fig. 9 shows fluorescence images at 540 nm and a pseudo real color image at 500 to 720 nm. b of Fig. 9 shows fluorescence images at 640 nm and a pseudo real color image at 600 to 820 nm. The exposure time (in milliseconds) of the fluorescence image is indicated at the bottom of each image.
c of Fig. 9 shows a composite image of green and red fluorescence images 30 minutes after administration of both probes to the breast cancer and benign tumor tissues. The exposure time is 40 milliseconds for the green image and 20 milliseconds for the red image. d of Fig. 9 shows comparison of the composite images at each time point.

From c of Fig. 9, the glycosidase-reactive fluorescent probe 5 emitted green fluorescence in the benign tumor, whereas the red GGT probe gGlu-20ME-SiR600 emitted red fluorescence in both breast cancer (malignant tumor) and the benign tumor. When the two images were combined, it was possible to image the breast cancer tissue in red and the benign tumor tissue in yellow. Therefore, by using the glycosidase-reactive fluorescent probe and the GGT probe in combination, it becomes possible to determine that a site where only the glycosidase-reactive fluorescent probe fluoresces is a benign tumor site, and a site where both the glycosidase-reactive fluorescent probe and the GGT probe fluoresce is a breast cancer (malignant tumor) site, and it becomes possible to distinguish between the benign tumor and the malignant tumor. Thus, the efficiency of a partial resection surgery can be improved without performing resection of the benign tumor site in a surgical procedure.

## Claims

1. A fluorescent probe for detecting breast cancer and a benign tumor of a mammary gland, said fluorescent probe comprising:
a compound represented by the following General Formula (I) or a salt thereof, 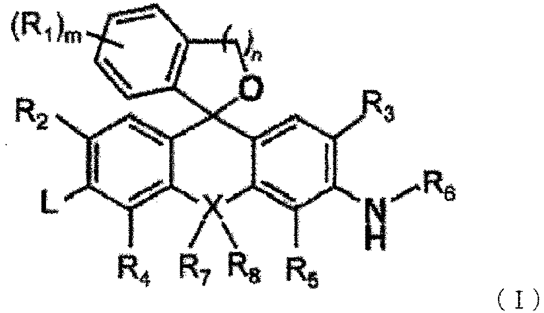 (In the formula,
R₁, if present, represents the same or different monovalent substituents present on a benzene ring;
R₂ and R₃ each independently represent a hydrogen atom, an alkyl group having 1 to 6 carbon atoms, or a halogen atom;
R₄ and R₅ each independently represent a hydrogen atom, an alkyl group having 1 to 6 carbon atoms, or a halogen atom;
R₆ represents a hydrogen atom, an alkyl group having 1 to 5 carbon atoms, or an alkyl fluoride group having 1 to 5 carbon atoms; and
R₇ and R₈, if present, each independently represent an alkyl group having 1 to 6 carbon atoms or an aryl group,
where R₇ and R₈ are not present in a case where X is an oxygen atom;
X represents an oxygen atom, a silicon atom, or a carbon atom;
n is an integer of 1 to 3; and
L is selected from any groups of the following Formulas (1) to (6)),

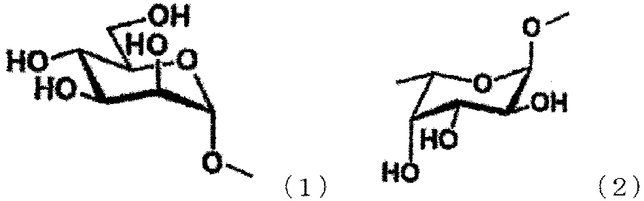

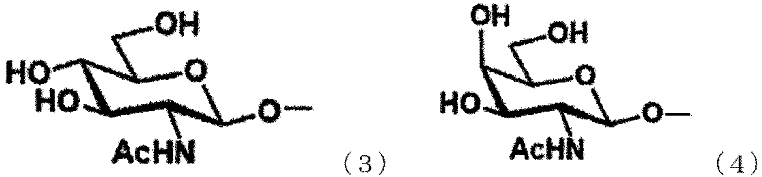

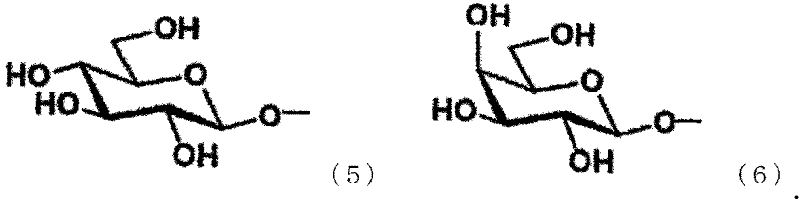

2. The fluorescent probe according to claim 1, wherein X is an oxygen atom.

3. The fluorescent probe according to claim 1 or 2, wherein R₆ is an alkyl fluoride group having 1 to 5 carbon atoms.

4. A fluorescent probe for detecting breast cancer and a benign tumor of a mammary gland, said fluorescent probe comprising: the fluorescent probe according to any one of claims 1 to 3; and a GGT-activated detection probe.

5. A fluorescent probe for detecting lung adenocarcinoma or lung squamous cell carcinoma, said fluorescent probe comprising: a compound represented by the following General Formula (I) or a salt thereof, 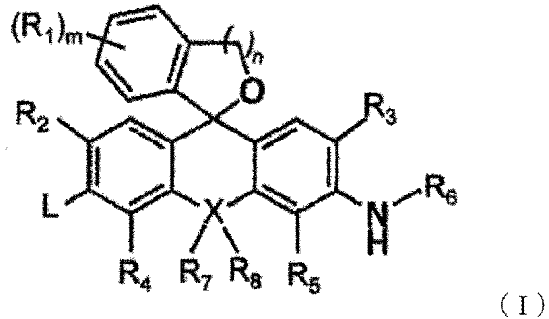 (In the formula,
R₁, if present, represents the same or different monovalent substituents present on a benzene ring;
R₂ and R₃ each independently represent a hydrogen atom, an alkyl group having 1 to 6 carbon atoms, or a halogen atom;
R₄ and R₅ each independently represent a hydrogen atom, an alkyl group having 1 to 6 carbon atoms, or a halogen atom;
R₆ represents a hydrogen atom, an alkyl group having 1 to 5 carbon atoms, or an alkyl fluoride group having 1 to 5 carbon atoms; and
R₇ and R₈, if present, each independently represent an alkyl group having 1 to 6 carbon atoms or an aryl group,
where R₇ and R₈ are not present in a case where X is an oxygen atom;
X represents an oxygen atom, a silicon atom, or a carbon atom;
n is an integer of 1 to 3; and
L is selected from groups of the following Formula (5) or (6)),

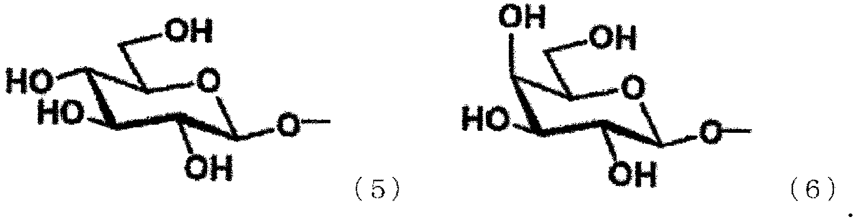

6. The fluorescent probe according to claim 5, wherein X is an oxygen atom.

7. The fluorescent probe according to claim 5 or 6, wherein R₆ is an alkyl fluoride group having 1 to 5 carbon atoms.

8. A method for detecting breast cancer and a benign tumor of a mammary gland, said method comprising: (a) a step of applying the fluorescent probe according to any one of claims 1 to 4 to a clinical breast specimen; and (b) measuring a fluorescence image of the clinical breast specimen to which the fluorescent probe is applied.

9. A method for detecting lung fiber cancer or lung squamous cell carcinoma, said method comprising: (a) a step of applying the fluorescent probe according to any one of claims 5 to 7 to a clinical lung fiber cancer or lung squamous cell carcinoma specimen; and (b) measuring a fluorescence image of the clinical lung fiber cancer or lung squamous cell carcinoma specimen to which the fluorescent probe is applied.

10. A fluorescent probe for detecting breast cancer and a benign tumor of a mammary gland, said fluorescent probe comprising: the fluorescent probe according to any one of claims 1 to 4; and a GGT-activated detection probe.

11. The fluorescent probe for detecting breast cancer and a benign tumor of a mammary gland according to claim 10, wherein the GGT-activated detection probe is a compound represented by the following General Formula (II) or a salt thereof, 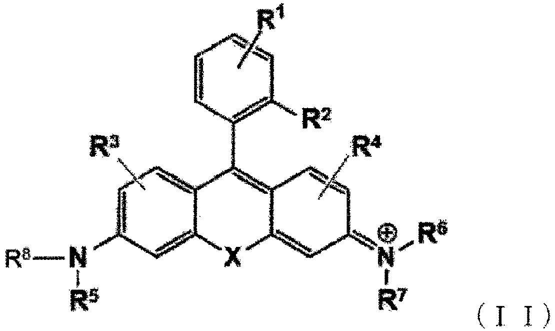 (In the formula,
X represents Si (R^{a}) (R^{b}), Ge (R^{a}) (R^{b}), Sn(R^{a}) (R^{b}), C (R^{a}) (R^{b}), P (=O) (R^{a}), or O,
wherein R^{a} and R^{b} each independently represent a hydrogen atom, an alkyl group, or an aryl group;
R¹ represents a hydrogen atom or 1 to 4 same or different substituents independently selected from the group consisting of an alkyl group, a carboxyl group, an ester group, an alkoxy group, an amide group, and an azide group, which may each be substituted;
R² represents a hydrogen atom, a hydroxyl group, a cyano group, or an alkyl group, an alkoxy group, an aryl, or a heteroaryl, which may each be substituted;
R³ and R⁴ each independently represent a hydrogen atom or 1 to 3 same or different substituents independently selected from the group consisting of a hydroxyl group, a halogen atom, and an alkyl group, a sulfo group, a carboxyl group, an ester group, an amide group, and an azide group, which may each be substituted; and
R⁵, R⁶, and R⁷ each independently represent a hydrogen atom or an alkyl group,
wherein R⁶ or R⁷ may each join with R⁴ to form a ring structure containing a nitrogen atom to which R⁶ and R⁷ are bonded; and
R⁸ represents an acyl residue derived from an amino acid).

12. A fluorescent probe for detecting breast cancer and a benign tumor of a mammary gland, said fluorescent probe comprising: the fluorescent probe according to any one of claims 1 to 4; and a compound of the following Formula (7) or a salt thereof,

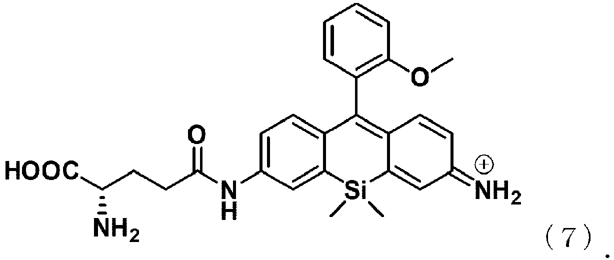

13. A kit for detecting breast cancer and a benign tumor of a mammary gland, said kit comprising: the fluorescent probe according to any one of claims 1 to 4.

14. A kit for detecting breast cancer and a benign tumor of a mammary gland, said kit comprising: the fluorescent probe according to any one of claims 1 to 4; and a GGT-activated detection probe.

15. A kit for detecting lung fiber cancer or lung squamous cell carcinoma, said kit comprising: the fluorescent probe according to any one of claims 5 to 7.
